# EUROPÄISCHE PATENTSCHRIFT

(11) **EP 0 626 070 B1**
(45) Veröffentlichungstag und Bekanntmachung des Hinweises auf die Patenterteilung: **03.04.1996**
(21) Anmeldenummer: 93903232.2
(22) Anmeldetag: 25.01.1993
(51) Int. Cl.: G01N 33/86, C12Q 1/56

(54) **VERFAHREN ZUR BESTIMMUNG VON HIRUDIN UND SYNTHETISCHEN TRHOMBININHIBITOREN**
METHOD OF DETERMINING HIRUDIN AND SYNTHETIC THROMBIN INHIBITORS
PROCEDE DE DETERMINATION DE L'HIRUDINE ET DES INHIBITEURS SYNTHETIQUES DE LA THROMBINE

(30) Priorität: 11.02.1992 DE 4203980
(43) Veröffentlichungstag der Anmeldung: 30.11.1994
(73) Patentinhaber: Max-Planck-Gesellschaft zur Förderung der Wissenschaften e.V., D-37073 Göttingen (DE)
(72) Erfinder: NOWAK, Götz, D-5083 Erfurt (DE); BUCHA, Elke, D-5032 Erfurt (DE); HOFFMANN, Jutta, D-5080 Erfurt (DE)
(74) Vertreter: Weisert, Annekäte, Dipl.-Ing. Dr.-Ing.
(86) Internationale Anmeldenummer: EP9300161
(87) Internationale Veröffentlichungsnummer: WO9316390

(56) Entgegenhaltungen:
- EP-A- 0 049 877
- EP-A- 0 420 332
- WO-A-92/07954
- JOURNAL OF BIOLOGICAL CHEMISTRY, Band 266, Nr. 6, 25 Februar 1991, Baltimore, MD (US); P.N.M. TIJBURG et al., Seiten 4017-4022/
- CHEMICAL ABSTRACTS, vol. 98, no. 3, 17 Januar 1983, Columbus, OH (US); E. BRIET et al., Seite 250, Nr. 13567/

## Beschreibung

Die Erfindung betrifft ein Verfahren zur Bestimmung von Hirudin und synthetischen Thrombininhibitoren im Blut sowie ein Mittel zur Durchführung des Verfahrens.

Hirudin, das aus der Speicheldrüse von Hirudo medicinalis (Blutegel) gewonnen wird, ist ein Antikoagulans, dessen Wirkung auf der Bildung einer chemischen Verbindung mit Thrombin beruht, wodurch dessen katalytische Wirkung inhibiert wird. Hirudin ist ein Miniprotein aus 65 Aminosäuren mit einem Molekulargewicht von 7 kD. Aufgrund seiner starken Affinität zu Thrombin (kᵢ-Werte von 10⁻¹ Mol/l) und seines direkten Wirkungsmechanismus ist es von großem Interesse. Seine klinische Anwendung war in der Vergangenheit äußerst beschränkt, da Hirudin nicht leicht in standardisierter Form zugänglich war. Hirudin kann heute gentechnologisch hergestellt werden und daher ist seine klinische Anwendung in der nächsten Zeit zu erwarten.

Beispielsweise werden in der EP-A-0 468 327 pharmazeutische Präparate für die orale Verabreichung beschrieben, die rekombinantes Hirudin enthalten.

Hirudin wurde in den letzten Jahren pharmakologisch intensiv untersucht und die pharmakologischen Daten wurden bei Versuchstieren und am Menschen erfaßt. Hirudin wird in der Leber nicht metabolisiert, sondern in unveränderter Form über die Nieren ausgeschieden. Hirudin hat eine Eliminationshalbwertszeit von etwa 1 bis 2 Stunden und verteilt sich in den extrazellulären Flüssigkeitsräumen des Körpers. Ähnlich wie Heparin wird Hirudin nicht oral resorbiert. In früheren Untersuchungen wurde gezeigt, daß Hirudin in nahezu allen Thrombosemodellen wirksam ist, so auch beim Endotoxinschock und bei experimenteller Koronarthrombose sowie bei der Verhinderung der Reocclusion nach Thrombolyse. Bei klinischpharmakologischen Untersuchungen wurden keine immunologischen Reaktionen nachgewiesen. Hirudin hat sich bei klinischen Untersuchungen als Antikoagulans und Antithrombotikum als dem Heparin überlegen erwiesen.

Augenblicklich wird weltweit in vielen Forschungslaboratorien zu synthetischen, insbesondere kleinmolekularen, Inhibitoren Synthesearbeit geleistet. Die synthetischen Thrombininhibitoren wirken auf gleiche Weise wie Hirudin. Am weitesten fortgeschritten sind Untersuchungen mit Derivaten des Benzamidins, wie zum Beispiel mit NAPAP (Nα-(2-Naphthylsulforyl-glycyl)-D,L-amidinophenylalanin-piperidid) und mit sogenannten Tripeptiden. Alle synthetischen Thrombininhibitoren befinden sich augenblicklich in der präklinischen Untersuchung. Ihre Wirkungen sind qualitativ denen von Hirudin gleichzusetzen. Der Metabolismus der synthetischen Thrombininhibitoren unterscheidet sich jedoch von dem des Hirudins. In der Regel werden die Thrombininhibitoren in der Leber oder im Blut metabolisiert. Es ist abzusehen, daß bereits in Kürze derartige Substanzen für die klinische Erprobung zur Verfügung stehen. Der Vorteil gegenüber dem Hirudin ist darin zu sehen, daß die Verbindungen oral verabreicht werden können.

Zu einer sachgerechten Therapie oder Prophylaxe ist es jedoch erforderlich, daß der Gehalt an Hirudin und synthetischen Thrombininhibitoren im Blut laufend bestimmt werden kann, um Unterdosierungen zu vermeiden oder Nebenwirkungen durch Überdosierungen zu verhindern. Mit anderen Worten, es muß ein therapeutisches drug monitoring verfügbar sein. Bis jetzt sind keine Verfahren zur Bestimmung von Hirudin und synthetischen Thrombininhibitoren, insbesondere im Blut, bekannt, die auf einfache Weise durchgeführt werden können.

Der vorliegenden Erfindung liegt die Aufgabe zugrunde, ein Verfahren zur Bestimmung von Hirudin und synthetischen Thrombininhibitoren, insbesondere im Blut, zur Verfügung zu stellen, das auf einfache Art und weise in Krankenhäusern, Arztpraxen und Laboratorien im breiten Umfang angewendet werden und beispielsweise bei der präoperativen Untersuchung zur Erkennung eines Blutungs- oder Thromboserisikos, zur Überwachung der Antikoagulantientherapie bei Thrombose gefährdeten Patienten und bei der Verlaufskontrolle zahlreicher Erkrankungen, zum Beispiel schwerer Infektionen, Leberfunktionsschäden und maligner Erkrankungen, verwendet werden kann.

Das erfindungsgemäße Verfahren soll auf leichte Weise durchgeführt werden können, wobei die Geräte verwendet werden sollen, die bereits in Krankenhäusern und Arztpraxen vorhanden sind. Außerdem soll ein Mittel zur Durchführung des Verfahrens zur Verfügung gestellt werden.

Gegenstand der Erfindung ist ein Verfahren zur Bestimmung von Hirudin oder synthetischen Thrombininhibitoren im Blut, das dadurch gekennzeichnet ist, daß zu dem Blut, dem Blutbestandteil oder der Körperflüssigkeit ein Prothrombin-Intermediat, eine Verbindung, die Prothrombin zu Meizothrombin spaltet, ein Salz davon oder ein Gemisch dieser Verbindungen sowie gegebenenfalls Puffer und/oder andere übliche Zusatzstoffe gegeben werden und die Zeit, die von der Zugabe bis zum Beginn der Gerinnung vergeht, gemessen wird, wobei als Prothrombin-Intermediat Meizothrombin, PIVKA-Meizothrombin oder Meizothrombin-des-Fragment-1 verwendet wird.

Gegenstand der Erfindung ist weiterhin ein Mittel zur Durchführung des oben genannten Verfahrens, das dadurch gekennzeichnet ist, daß es ein vorstehend definiertes Prothrombin-Intermediat, eine Verbindung, die Prothrombin zu Meizothrombin spaltet, ein Salz davon oder ein Gemisch dieser Verbindungen zusammen mit Puffern und/oder anderen üblichen Zusatzstoffen enthält.

Das erfindungsgemäße Verfahren besitzt den großen Vorteil, daß es sehr einfach durchzuführen ist und daß die Ergebnisse des Untersuchungstests schnell verfügbar sind, da sie an Eichkurven abgelesen werden können. Das Verfahren kann auf einfache Weise in Krankenhäusern, Arztpraxen und Laboratorien im breiten Umfang durchgeführt werden und erfordert kein besonders ausgebildetes Personal.

Das erfindungsgemäße Verfahren kann zur Bestimmung von Hirudin oder synthetischen, vorzugsweise kleinmolekularen, Thrombininhibitoren durchgeführt werden. Beispiele für synthetische Thrombininhibitoren sind vor allem die Derivate des Tripeptids Phe-Pro-Arg, wie Boronsäure-Derivate, Argininale, Chlormethylketon-Derivate und Derivate, die an den Aminosäuren modifiziert sind sowie Benzamidin-Derivate und auch sogenannte Hirologe, d.h. synthetische Hirudin-analoge Teilsequenzen. Das Antidotprinzip ist das gleiche wie bei Hirudin.

Der wissenschaftliche Hintergrund dieser Methode beruht auf der Interaktion von Hirudin oder synthetischen Thrombininhibitoren mit einem Prothrombin-Intermediat, wie intermediärem Meizothrombin, einem Zwischenprodukt der Prothrombin-Thrombin-Umwandlung, oder Meizothrombin-des-Fragment-1. Es handelt sich bei dieser Umwandlung um eine mehrstufige Reaktion, wobei ganz spezielle Proteinbindungen durch limitierte proteolytische Reaktionsschritte gelöst werden. Normalerweise wird bei der Aktivierung der Gerinnungskaskade ein proteolytisch wirksamer Komplex aus aktiviertem Faktor V, Ca⁻⁻, Phospholipid und Faktor X gebildet, der die Prothrombin-Thrombin-Umwandlung initiiert. Dabei entstehen nur geringe Quantitäten von Intermediaten des Faktors II, weil die Prothrombin-Aktivierung an diesem Prothrombinase-Komplex quasi "festphasenartig" erfolgt. Wird die Gerinnung jedoch mit einer spezifischen Schlangengiftfraktion, z.B. aus Echiscarinatus, eingeleitet, dann werden überwiegend "atypische" Intermediate, z.B. Meizothrombin, PIVKA-Meizothrombin oder Meizothrombin-des-Fragment-1, aus Prothrombin gebildet. Diese Intermediate werden durch Hirudin oder synthetische Thrombininhibitoren inaktiviert, nicht aber durch Heparin. Somit ist es möglich, einen Gerinnungstest aufzubauen, bei dem im Blut oder in einem Blutbestandteil durch ein Schlangengift, beispielsweise Echis-Schlangengift, Meizothrombin oder PIVKA-Meizothrombin erzeugt wird, welches durch das in der Probe enthaltene Hirudin inaktiviert wird. Die Affinität des Hirudins und der synthetischen Thrombininhibitoren zu den Prothrombin-Intermediaten ist sehr hoch. Sie beträgt zum Beispiel für Meizothrombin kᵢ > 10⁻¹⁰ mol/l, so daß erst nach vollständigem Verbrauch des Hirudins oder der synthetischen Thrombininhibitoren in der Probe freies Prothrombin-Intermediat, beispielsweise Meizothrombin, entsteht. Dieses Prothrombin-Intermediat, zum Beispiel das Meizothrombin, kann das in der Probe enthaltene Fibrinogen zu Fibrin umwandeln. Diese Fibrinbildung wird durch das Gerinnen der Probe zeitlich dokumentiert. Die Quantifizierung ist dadurch gegeben, daß der Gehalt der Blutprobe an Hirudin die Gerinnungszeit im therapeutischen Blutspiegelbereich linear verlängert und somit eine rasche und problemlose Aussage zuläßt.

Bei dem erfindungsgemäßen Verfahren wird die Zeit, die von der Zugabe der Verbindung zu dem Blut bis zum Gerinnungseintritt vergeht, gemessen. Für die Messung des Gerinnungseintritts sind zahlreiche Methoden bekannt. Am häufigsten wird in den Gerinnungsansatz periodisch ein Platin-Häkchen eingeführt, welches bei der Bildung eines Gerinnsels einen Fibrinfaden aus der Lösung herauszieht. Dieser Zeitpunkt wird dann als Gerinnungsendpunkt registriert. Der Registrierungsvorgang kann manuell mittels Stoppuhr oder elektrisch durch Auslösung eines Kontaktes, wenn das Platin-Häkchen als Elektrode ausgelegt ist, erfolgen. Weiterhin gibt es automatisierte Methoden.

Das erfindungsgemäße Verfahren besitzt den großen Vorteil, daß die Adaptierung auf alle Meßprinzipien der Gerinnungsdiagnostik, besonders auch auf automatisierte Methoden möglich ist. Bei Zugabe von chromogenen Thrombinsubstraten zu entsprechenden Plasmaproben ist eine Meßdurchführung in Laborautomaten möglich.

Das erfindungsgemäße Verfahren kann auf einfache Weise durchgeführt werden und erlaubt die Ablesung des Hirudingehalts an einer Eichkurve. Das erfindungsgemäße Verfahren kann mit Gesamtblut, Blutplasma, aber auch mit Körperflüssigkeiten, wie Urin, Gewebepressaft oder Zelleluaten nach Homogenisierung, zu denen eine Quantität Normalplasma gegeben wird, durchgeführt werden. Das erfindungsgemäße Verfahren hat den großen Vorteil, daß das Blut noch Heparin enthalten kann, da Heparin das Verfahren nicht stört.

Erfindungsgemäß wird als Prothrombin-Intermediat beispielsweise Meizothrombin, PIVKA-Prothrombin oder Meizothrombin-des-Fragment-1 verwendet. Meizothrombin ist im Handel erhältlich und kann von der Firma Pentapharm in der Schweiz bezogen werden. Meizothrombin, PIVKA-Prothrombin oder andere Prothrombin-Intermediate können aber auch in vitro gebildet werden.

Vier Faktoren des Gerinnungssystems, Faktor II (Prothrombin), Faktor VII, Faktor IX und Faktor X sind, wie im folgenden Schema dargestellt, dadurch gekennzeichnet, daß sie gamma-Carboxyglutaminsäurereste enthalten. Diese gamma-Carboxylierung an der Glutaminsäure erfolgt erst nach der ribosomalen Synthese des "Acarboxy-Faktors" in der Leber mit Hilfe eines Enzymsystems, welches Vitamin K als Cofaktor benötigt.

Die gamma-Carboxyglutaminsäurereste sind für die Gerinnungswirkung essentiell. Sie stellen die nötigen Bindungsvalenzen für Calciumionen dar. Bei Behandlung mit indirekten Antikoagulantien vom Typ der Dicumarole ("Vitamin-K-Antagonisten") kann die postribosomale gamma-Carboxylierung nicht stattfinden und es befinden sich im Blut inkomplette Gerinnungsfaktoren bzw. Acarboxy-Faktoren, denn ihnen fehlen die Calcium-bindenden gamma-Carboxy-Gruppen. Diese Gerinnungsfaktoren werden auch PIVKA-Faktoren (PIVKA = proteins induced by Vitamin K antagonists) genannt.

Wenn Plasma von Patienten, die mit derartigen Antikoagulantien vom Dicumarol-Typ behandelt wurden, mit Ecarin versetzt wird, dann entsteht in diesem Plasma aus dem PIVKA-Prothrombin PIVKA-Meizothrombin in der gleichen Weise durch eine limitierte Proteolyse wie dies auch in normalen Plasmaproben mit Prothrombin geschieht.

Dieses PIVKA-Meizothrombin bzw. andere PIVKA-Intermediate haben ihre Bindungsfähigkeit zu Hirudin behalten, sie haben aber keine bzw. wesentlich geringere Wirkungen auf andere Faktoren der Gerinnungskaskade (Plättchen, Fibrinogen, Thrombomodulin u.a.). Erfindungsgemäß können Meizothrombin, PIVKA-Meizothrombin, deren Intermediate und PIVKA-Intermediate aus PIVKA-Prothrombin verwendet werden. Diese können vom Menschen oder von anderen Säugetieren stammen.

Zur Herstellung von Meizothrombin kann beispielsweise immobilisiertes Ecarin in Minisäulen in einer Größe von beispielsweise 2 - 4 cm³ gepackt werden. Ecarin-Immobilisat (Pentapharm AG, Basel) gelangt in gequollenem Zustand, suspendiert in einer wäßrigen Lösung von Natriumchlorid 0,15 M, Natriumacetat 0,02 M, Prionex® (Warenzeichen der Pentapharm AG für eine proteinstabilisierende Polypeptidfraktion aus gereinigtem Schweinehaut-Kollagen) 0,2% und Trichlorisobutanol 0,3%, pH 5,5, zur Auslieferung. 1 g gequollenes Ecarin-Immobilisat produziert aus Bariumcitrat-Eluat bei 37°C, pH 8,4, innerhalb von 30 min 500 bis 700 U amidolytische Aktivität (1 U = 123 NIH-Einheiten), gemessen an Tos-Gly-Pro-Arg-pNA (Chromozym® TH).

Dann werden gereinigte Prothrombinfraktionen auf diese Säulen gegeben, und das gebildete Meizothrombin, gegebenenfalls nach Stabilisierung mit Heparin, wird anschließend gefriergetrocknet. Das gefriergetrocknete Material kann in Ampullen abgepackt werden und dann für die Verwendung mit einem geeigneten Lösungsmittel rekonstituiert werden.

Zur Herstellung von Meizothrombin-des-Fragment-1 wird dasselbe Verfahren wie für Meizothrombin verwendet. Im Batch-Verfahren ist nur eine längere Reaktionszeit (3 - 4 h) vorzusehen. Meizothrombin-des-Fragment-1 ist ein Folgeprodukt der Meizothrombin-Aktivierung.

Erfindungsgemäß wird als Verbindung, die Prothrombin zu Meizothrombin spaltet, ein Schlangengift verwendet. Beispiele für Schlangengifte sind Ecarin und die Gifte von Dispholidus-, Rhabdophis-, Bothrops-, Notechis-, Oxyuranus- und Russell-Vipern-Arten. Die Schlangengifte, wie Ecarin sowie immobilisiertes Ecarin, sind im Handel erhältlich und können beispielsweise von der Firma Pentapharm in der Schweiz käuflich bezogen werden.

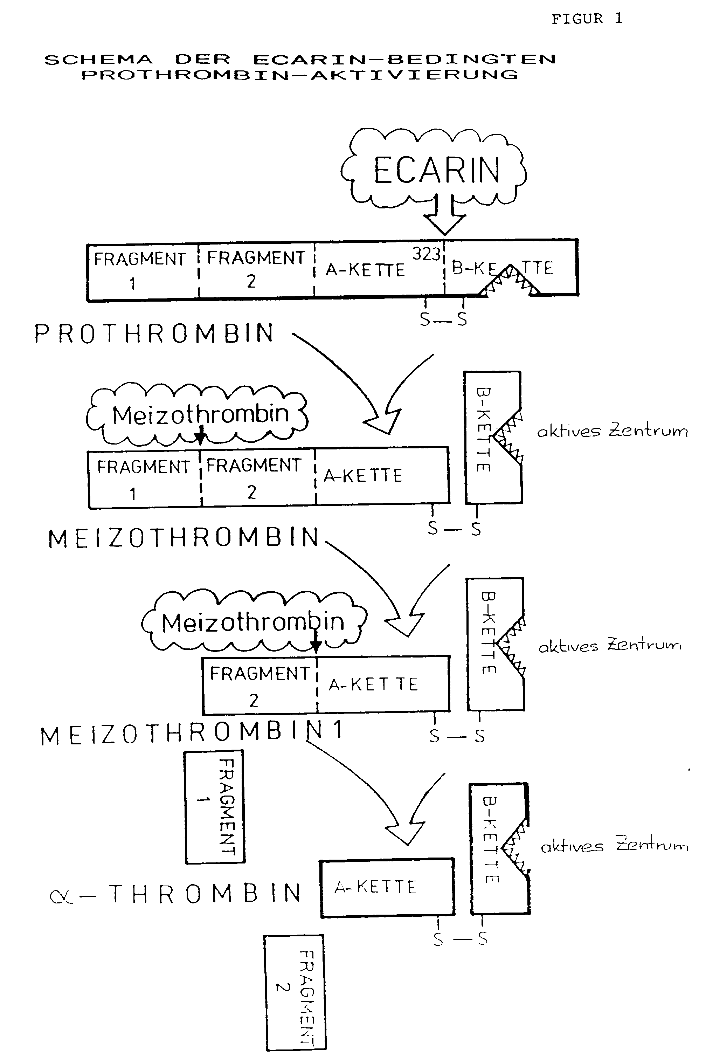

Erfindungsgemäß wird als Schlangengift bevorzugt Ecarin, eine hochgereinigte Fraktion des Echis-carinatus-Toxins, verwendet. Ecarin spaltet am Arginin 323 des Prothrombins eine Peptidbindung, wodurch das Intermediat Meizothrombin entsteht. Normalerweise erfolgt die weitere Umsetzung durch Autokatalyse bzw. durch Thrombinakzeleration. Bei Vorliegen von Hirudin im Plasma kommt es zur Interaktion zwischen Meizothrombin und Hirudin. Im Gegensatz dazu kann Heparin nicht mit Meizothrombin reagieren. Diese Wirkungen sind in der beigefügten Figur 1 dargestellt.

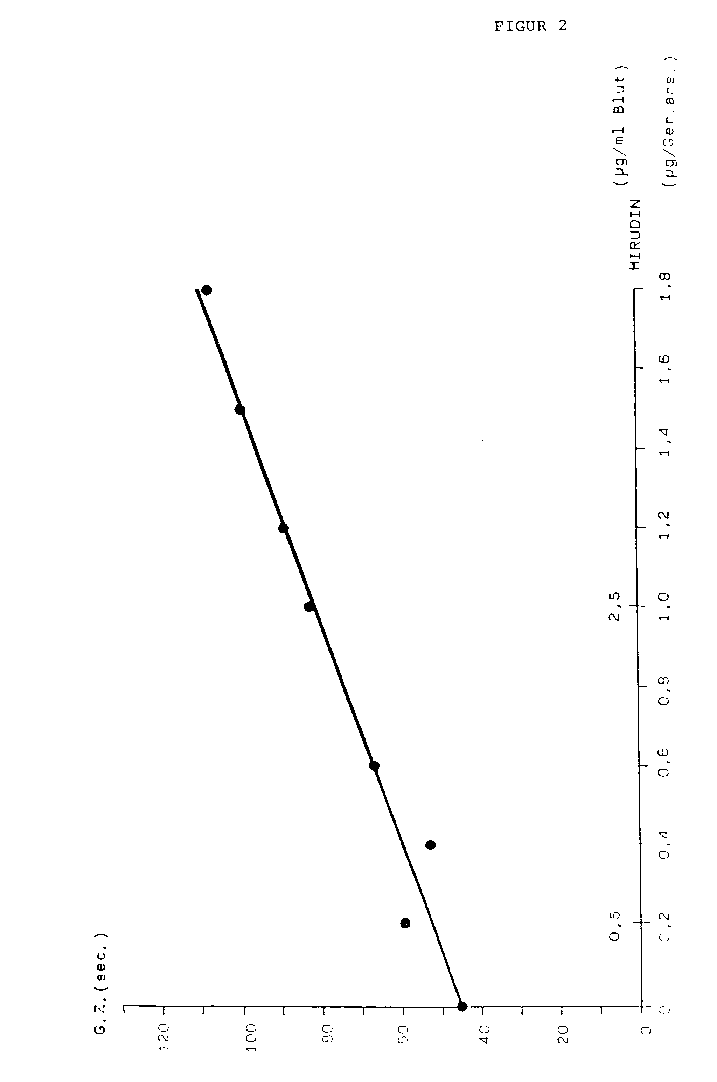

Zur Bestimmung des Hirudingehalts im Blut wird wie folgt vorgegangen: Zu 0,4 ml Heparinblut (20 IE Heparin/ml Blut) werden 0,16 ml Puffer, 0,02 ml 0,1 M CaCl₂-Lösung und 0,02 ml Ecarin (200 EU/ml) gegeben. Der Gerinnungseintritt wird mittels mechanischer automatischer Gerinnungszeitmessung bestimmt. Zur Erstellung einer Eichkurve werden in den Pufferanteil des Gerinnungsansatzes steigende Mengen Hirudin gegeben. Aus der beigefügten Figur 2 ist ersichtlich, daß über einen Konzentrationsbereich von 0,1 - 2 µg Hirudin/Gerinnungsansatz eine Linearität besteht. Dadurch sind sehr schnell Aussagen zum Hirudingehalt einer Blutprobe möglich. Ein weiterer Vorteil besteht in der Verwendung von Vollblut. Die gesamte Meßzeit ist mit etwa 5 min optimal für ein therapeutisches drug monitoring, im Sinne einer bed-side-Diagnostik.

Das folgende Beispiel erläutert die Erfindung.

In dem nachfolgenden Testbeispiel wurde ein Gerinnungszeit-Meßgerät benutzt, bei dem mittels Magnet im Gerinnungsansatz der Gerinnungseintritt durch Störung eines elektrischen Feldes bei rotierenden Probenröhrchen erfaßt wird. Dadurch ist Vollblut als Probenansatz zu benutzen. Der "Blood Coagulation Timer" Hemochron® Modell 801 der Int. Technidyne Corp. Edison, N.J., USA, verfügt über zwei Testkanäle und ermöglicht dadurch problemlos eine Doppelbestimmung.

### BEISPIEL

Im Hemochron-Teströhrchen vom Typ P214 wurden jeweils
0,02 ml CaCl₂ 0,1 M
0,02 ml Ecarin 200 EU/ml NaCl
0,16 ml Tris-Puffer 0,05 M pH 7,4 bei 37°C
0,40 ml Testblut
rasch gemischt, die Zeit gestartet, das Teströhrchen in den Gerinnungsautomaten eingesetzt und die Zeit für den Gerinnungseintritt registriert. In gleicher Weise wird verfahren, um eine Eichkurve aufzustellen, aus der man die Hirudinkonzentration bei entsprechender Gerinnungszeit ablesen kann. Dabei werden dem Puffer definierte Hirudinmengen und statt Testblut unbehandeltes Blut zugesetzt. Die Gerinnungszeit verlängert sich mit steigender Hirudinkonzentration.

Bei vier Probanden wurden durch Doppelbestimmung folgende Werte ermittelt:

| | Gerinnungszeit [sec] | Hirudin/Ger.-ansatz [µg] | Huridin/ml Blut [µg] |
|---|---|---|---|
| Prob. A | (70; 63) φ 66,5 | 0,5 | 1,25 |
| Prob. B | (79; 86) φ 82,5 | 0,9 | 2,25 |
| Prob. C | (46; 50) φ 48,0 | 0,1 | 0,25 |
| Prob. D | (44; 48) φ 46,0 | 0,0 | 0,00 |

Es ist erkennbar, daß bei Prob. D eine Unterdosierung bzw. ein Hirudinverbrauch vorliegt. Bei Prob. C und A befindet sich der Hirudinblutspiegel im therapeutischen Bereich und bei Prob. B im toxischen Bereich.

## Patentansprüche

1. Verfahren zur Bestimmung von Hirudin und synthetischen Thrombininhibitoren im Blut, in Blutbestandteilen oder in Körperflüssigkeiten, dadurch **gekennzeichnet**, daß zu dem Blut, dem Blutbestandteil oder der Körperflüssigkeit ein Prothrombin-Intermediat, eine Verbindung, die Prothrombin zu Meizothrombin spaltet, ein Salz davon oder ein Gemisch dieser Verbindungen sowie gegebenenfalls Puffer und/oder andere übliche Zusatzstoffe gegeben werden und die Zeit, die von der Zugabe bis zum Beginn der Gerinnung vergeht, gemessen wird, wobei als Prothrombin-Intermediat Meizothrombin, PIVKA-Meizothrombin oder Meizothrombin-des-Fragment-1 verwendet wird.

2. Verfahren nach Anspruch 1, dadurch **gekennzeichnet**, daß das Blut oder der Blutbestandteil Heparin enthält.

3. Verfahren nach Anspruch 1 oder 2, dadurch **gekennzeichnet**, daß als Prothrombin-Intermediat Meizothrombin, PIVKA-Meizothrombin oder Meizothrombin-des-Fragment-l und als Verbindung, die Prothrombin zu Meizothrombin spaltet, ein Schlangengift verwendet wird.

4. Verfahren nach Anspruch 3, dadurch **gekennzeichnet**, daß als Schlangengift Ecarin verwendet wird.

5. Mittel zur Durchführung des Verfahrens nach einem der Ansprüche 1 bis 4, dadurch **gekennzeichnet**, daß es ein Prothrombin-Intermediat, eine Verbindung, die Prothrombin zu Meizothrombin spaltet, ein Salz davon oder ein Gemisch dieser Verbindungen zusammen mit Puffern und/oder anderen üblichen Zusatzstoffen enthält, wobei es als Prothrombin-Intermediat Meizothrombin, PIVKA-Meizothrombin oder Meizothrombin-des-Fragment-1 und als Verbindung, die Prothrombin zu Meizothrombin spaltet, ein Schlangengift enthält.

6. Mittel nach Anspruch 5, dadurch **gekennzeichnet**, daß es als Schlangengift Ecarin enthält.

7. Verwendung eines Mittels, enthaltend ein Prothrombin-Intermediat, eine Verbindung, die Prothrombin zu Meizothrombin spaltet, ein Salz davon oder ein Gemisch dieser Verbindungen zur Bestimmung von Hirudin und synthetischen Thrombininhibitoren im Blut, in Blutbestandteilen oder in Körperflüssigkeiten.

8. Verwendung nach Anspruch 7, dadurch **gekennzeichnet**, daß das Mittel als Prothrombin-Intermediat Meizothrombin, PIVKA-Meizothrombin oder Meizothrombin-des-Fragment-1 und/oder als Verbindung, die Prothrombin zu Meizothrombin spaltet, ein Schlangengift enthält.

9. Verwendung nach Anspruch 8, dadurch **gekennzeichnet**, daß das Mittel als Schlangengift Ecarin enthält.

## Claims

1. Process for determining hirudin and synthetic thrombin inhibitors in the blood, blood constituents or body fluids, characterised in that a prothrombin intermediate, a compound which splits prothrombin into meizothrombin, a salt thereof or a mixture of these compounds optionally together with buffers and/or other conventional additives are added to the blood, the blood constituent or the body fluid and the time elapsing from the addition to the beginning of clotting is measured, wherein meizothrombin, PIVKA meizothrombin or meizothrombin des-fragment 1 is used as the prothrombin intermediate.

2. Process according to claim 1, characterised in that the blood or blood constituent contains heparin.

3. Process according to claim 1 or 2, characterised in that meizothrombin, PIVKA meizothrombin or meizothrombin des-fragment 1 is used as the prothrombin intermediate and a snake venom is used as the compound which splits prothrombin into meizothrombin.

4. Process according to claim 3, characterised in that ecarin is used as the snake venom.

5. Means for performing the process according to one of claims 1 to 4, characterised in that it contains a prothrombin intermediate, a compound which splits prothrombin into meizothrombin, a salt thereof or a mixture of these compounds together with buffers and/or other conventional additives, wherein it contains meizothrombin, PIVKA meizothrombin or meizothrombin des-fragment 1 as the prothrombin intermediate and snake venom as the compound which splits prothrombin into meizothrombin.

6. Means according to claim 5, characterised in that it contains ecarin as the snake venom.

7. Use of a means containing a prothrombin intermediate, a compound which splits prothrombin into meizothrombin, a salt thereof or a mixture of these compounds to determine hirudin and synthetic thrombin inhibitors in the blood, blood constituents or body fluids.

8. Use according to claim 7, characterised in that the means contains meizothrombin, PIVKA meizothrombin or meizothrombin des-fragment 1 as the prothrombin intermediate and/or snake venom as the compound which splits prothrombin into meizothrombin.

9. Use according claim 8, characterised in that the means contains ecarin as the snake venom.

## Revendications

1. Procédé de détermination de l'hirudine et d'inhibiteurs synthétiques de la thrombine dans le sang, dans les composants du sang ou dans des liquides corporels, caractérisé en ce qu'on ajoute au sang, au composant du sang ou au liquide corporel, un composé intermédiaire de la prothrombine, un composé qui décompose la prothrombine en meizothrombine, un sel de celui-ci ou un mélange de ces composés, ainsi qu'éventuellement des tampons et/ou d'autres additifs usuels, et on mesure le temps écoulé entre cette addition et le début de la coagulation, le composé intermédiaire de prothrombine qu'on utilise étant la meizothrombine, la PIVKA-meizothrombine ou la meizothrombine-des-fragment-1.

2. Procédé selon la revendication 1, caractérisé en ce que le sang ou le composant du sang contient de l'héparine.

3. Procédé selon la revendication 1 ou 2, caractérisé en ce qu'on utilise de la meizothrombine, de la PIVKA-meizothrombine ou de la meizothrombine-des-fragment-1 comme composé intermédiaire de la prothrombine, et un venin de serpent comme composé qui décompose la prothrombine en meizothrombine.

4. Procédé selon la revendication 3, caractérisé en ce qu'on utilise de l'écarine comme venin de serpent.

5. Agent pour la mise en oeuvre du procédé selon l'une des revendications 1 à 4, caractérisé en ce qu'il contient un composé intermédiaire de la prothrombine, un composé qui décompose la prothrombine en meizothrombine, un sel de celui-ci ou un mélange de ces composés, conjointement avec des tampons et/ou d'autres additifs usuels, ledit agent contenant de la meizothrombine, de la PIVKA-meizothrombine ou de la meizothrombine-des-fragment-1 comme composé intermédiaire de la prothrombine, et un venin de serpent comme composé qui décompose la prothrombine en meizothrombine.

6. Agent selon la revendication 5, caractérisé en ce qu'il contient de l'écarine comme venin de serpent.

7. Utilisation d'un agent contenant un composé intermédiaire de la prothrombine, un composé qui décompose la prothrombine en meizothrombine, un sel de celui-ci ou un mélange de ces composés, pour la détermination de l'hirudine et d'inhibiteurs synthétiques de la thrombine dans le sang, dans des composants du sang ou dans des liquides corporels.

8. Utilisation selon la revendication 7, caractérisée en ce que l'agent contient de la meizothrombine, de la PIVKA-meizothrombine ou de la meizothrombine-des-fragment-1 comme composé intermédiaire de la prothrombine et/ou un venin de serpent comme composé qui décompose la prothrombine en meizothrombine.

9. Utilisation selon la revendication 8, caractérisée en ce que l'agent contient de l'écarine comme venin de serpent.
